# EUROPÄISCHE PATENTSCHRIFT

(11) **EP 2 092 050 B1**
(45) Veröffentlichungstag und Bekanntmachung des Hinweises auf die Patenterteilung: **13.11.2013**
(21) Anmeldenummer: 07845299.2
(22) Anmeldetag: 19.12.2007
(51) Int. Cl.: C12M 1/09, C12M 1/12

(54) **VERFAHREN ZUR BEHANDLUNG EINES STOFFSTROMES**
METHOD FOR THE TREATMENT OF A MATERIAL FLOW
PROCÉDÉ DE TRAITEMENT D'UN FLUX DE SUBSTANCE

(30) Priorität: 19.12.2006 AT 20912006
(43) Veröffentlichungstag der Anmeldung: 26.08.2009
(73) Patentinhaber: Gruene-Bioraffinerie.At Gmbh, 8074 Raaba (AT)
(72) Erfinder: KOSCHUH, Werner, 8045 Graz (AT); KROMUS, Stefan, 1210 Wien (AT)
(74) Vertreter: Nemec, Harald
(86) Internationale Anmeldenummer: PCT/AT2007/000571
(87) Internationale Veröffentlichungsnummer: WO 2008/074042

(56) Entgegenhaltungen:
- EP-A- 0 049 055
- EP-A- 0 295 591
- EP-A- 0 531 864
- DE-A1- 3 215 818
- DE-A1- 3 812 440
- DE-A1- 4 427 478
- DE-C1- 4 212 187

## Beschreibung

Die vorliegende Erfindung betrifft ein Verfahren zur Behandlung eines komplexen Stoffstromes, der mehrere Wertstoffe verschiedener chemischer Natur enthält.

Die Problematik des Auftrennens oder Behandelns von komplexen Stoffströmen, um die darin enthaltenen verschiedenen Wertstoffe anzureichern, stellt sich insbesondere im Bereich von wässerigen Flüssigkeiten auf biogener Basis, wie z.B. Flüssigkeiten aus Fermentationsprozessen und insbesondere Flüssigkeiten, die aus einer Silage gewonnen werden.

Flüssigkeiten, die aus einer Ganzpflanzensilage oder einer Silage aus Grünlandbiomasse (wie z.B. aus Gras, Klee, Luzerne, Kräutern etc.) gewonnen werden, werden seit einiger Zeit als interessante Rohstoffe für die Gewinnung von Feinchemikalien angesehen. Im folgenden steht der Begriff "Gras-Silage" stellvertretend für sämtliche Silagen aus Grünlandbiomasse.

Im Rahmen des "Österreichischen Programmes zur Entwicklung der Green Biorefinery" wurde gezeigt, das die während des Silagevorganges von z.B. Gras anfallenden Wertstoffe wie Milchsäure und proteinogene Aminosäuren den biogenen Rohstoff Gras zu einer potentiell interessanten Quelle zur Gewinnung solcher Wertstoffe machen. Diese Idee ist insbesondere aufgrund der Möglichkeit der Verwendung eines billligen und noch dazu erneuerbaren Rohstoffes interessant.

Problematisch ist allerdings, dass die beispielsweise bei der Gras-Silage anfallenden komplexen Stoffströme eine Mehrzahl von Wertstoffen unterschiedlicher chemischer Natur aufweisen, deren Auftrennung schwierig ist.

Die vorliegende Erfindung beschäftigt sich insbesondere mit komplexen Stoffströmen, die jeweils zumindest einen Wertstoff der Gruppen
(A) Aminosäuren
(B) Von Aminosäuren unterschiedliche Carbonsäuren mit 1 bis 5 C-Atomen und
(C) Anorganische Salze
enthalten.

Mischungen dieser drei Gruppen von Wertstoffen sind insbesondere in den bereits erwähnten Flüssigkeiten aus einer Gras-Silage anzutreffen. Diese werden im folgenden zusammenfassend mit dem Begriff "Silageflüssigkeit" bezeichnet.

Hong Thang et al., J.Membr.Sc. 249 (2005) 173-182 beschreiben die Anwendung eines elektrodialytischen Verfahrens zur Abtrennung von Milchsäure aus einer komplexen Flüssigkeit aus einer Gras-Silage.

Koschuh et al., Desalination 163 (2004) 254-259 beschreiben die Anwendung einer Ultrafiltration an Flüssigkeiten aus gepresstem Gras und Alfalfa zur Gewinnung eines an Protein reichen Konzentrates.

Hong Thang et al., Desalination 162 (2004) 343-353 beschreiben ein zweistufiges Elektrodialyse-Verfahren zur Behandlung einer Gras-Silage. In der ersten Stufe dieses Verfahrens findet eine weitgehende Entsalzung des Stoffstromes statt, in der zweiten Stufe wird Milchsäure angereichert.

Koschuh et al., J.Membr.Sc. 261 (2005) 121-128 untersuchen das Fluss- und Retentionsverhalten von Nanofiltrations- und feinen Ultrafiltrationsmembranen bei der Filtration von Silage-Flüssigkeit.

Hong Thang et al., J.Membr.Sc. 256 (2005) 78-88 vergleichen Elektrodialyseverfahren mit chromatographischen Verfahren hinsichtlich ihrer Wirksamkeit zur Entsalzung von Silage-Flüssigkeit.

Im veröffentlichten Stand der Technik wurde bislang ausschließlich die Anreicherung von einer oder bestenfalls zwei der oben genannten Wertstoffgruppen (A), (B) und (C) beschrieben.

Die bekannten Verfahren sind zudem durch ein unscharfes Trennvermögen, insbesondere zwischen Aminosäuren (A) und organischen Säuren (B) gekennzeichnet. Durch eine Serie solcher Prozesschritte mit jeweils unscharfer Trennung kommt es nur zu geringen Ausbeuten, d.h. zu Nebenströmen mit geringen Konzentrationen des Wertstoffes. Solche Nebenströme können meistens nicht wirtschaftlich genutzt werden und sind demnach Abfall.

Wird z.B. eine solche Prozessserie mit Nanofiltrationsschritten durchgeführt, werden zusätzlich enorme Mengen von Prozesswasser für die Ausspülung der ungewünschten Begleitstoffe (B) und (C) benötigt.

Wird eine solche Prozessserie mit Elehtrodialyseverfahren durchgeführt, so muss die Elektrodialyse, um hohe Ausbeuten zur gewährleisten, zum Teil bei ungünstigen Wertstofflconzentrationen durchgeführt werden. Bei solch ungünstigen bzw. niedrigen Wertstoffkonzentrationen kommt es zu erhöhten Verlusten und ungewollten Transfer von Begleitstoffen.

Die vorliegende Erfindung stellt sich zur Aufgabe, ein Verfahren zur Verfügung zu stellen, mit welchem sämtliche in einem komplexen Stoffstrom, insbesondere einer Silage-Flüssigkeit enthaltenen Wertstoffgruppen (A), (B) und (C) in für eine Weiterverarbeitung ausreichender Konzentration angereichert werden können.

Diese Aufgabe wird mit einem Verfahren gemäß Anspruch 1 zur Behandlung eines Stoffstromes, der aus jeder der folgenden Gruppen
(A) Aminosäuren
(B) Von Aminosäuren unterschiedliche Carbonsäuren mit 1 bis 5 C-Atomen und
(C) Anorganische Salze
zumindest einen Wertstoff enthält, gelöst, welches die folgenden Stufen aufweist:
(1) Behandlung des Stoffstromes mittels Nanofiltration zur Gewinnung eines mit Wertstoff (A) angereicherten Retentates
(2) Behandlung des Permeates des Schrittes (1) mittels Elektrodialyse zur Gewinnung eines mit Wertstoff (C) angereicherten Konzentrates
(3) Behandlung des Diluates des Schrittes (2) mittels eines Systems (3) von zwei miteinander direkt oder indirekt zusammengeschalteten Stufen (4) und (5), wobei
(4) eine Behandlung mittels Umkehrosmose durchgeführt wird und
(5) eine Behandlung mittels Elektrodialyse durchgeführt wird, wobei ein mit Wertstoff (B) angereichertes Konzentrat gewonnen wird
(6) zumindest ein Teil des Retentates des Schrittes (4) direkt oder indirekt dem Schritt (5) zugeführt wird
(7) zumindest ein Teil des Diluates des Schrittes (5) direkt oder indirekt dem Schritt (4) zugeführt wird.

Es wird somit zunächst ein Nanofiltrationsschritt durchgeführt, bei welchem ein mit Wertstoff (A), also Aminosäuren, angereichertes Retentat erhalten wird. Dieses Retentat wird aus dem Gesamtprozess ausgeschleust.

Mittels eines unter entsprechenden Bedingungen betriebenen Elektrodialyse-Verfahrens kann aus dem Permeat der Nanofiltration des Schrittes (1) ein mit dem Wertstoff (C), d.h. den anorganischen Salzen angereichertes Konzentrat erzielt werden. Dieses Konzentrat wird ebenfalls aus dem Gesamtprozess ausgeschleust.

Das Diluat der Elektrodialyse (2) wird einem System (3) zugeführt, welches eine Behandlung mittels Umkehrosmose (5) und mittels Elektrodialyse (5) umfasst, wobei die Stufen (4) und (5) miteinander direkt oder indirekt verbunden sind.

Dabei wird zumindest ein Teil des Retentates der Schrittes (4) direkt oder indirekt dem Schritt (5) zugeführt und wird zumindest ein Teil des Diluates des Schrittes (5) direkt oder indirekt dem Schritt (4) zugeführt.

Mittels der Umkehrosmosebehandlung im Schritt (4) findet eine Aufkonzentrierung des Diluates des Schrittes (2) statt.

Bei der Elektrödialysebehandlung (5) wird unter entsprechenden Bedingungen ein mit Wertstoff (B), also den organischen Carbonsäuren angereichertes Konzentrat erhalten.

Durch eine direkte oder indirekte Rückführung des Diluates der Elektrodialyse des Schrittes (5) zur Umkehrosmose (Schritt (4)) wird eine ständige weitere Anreicherung der Wertstoffe (B) erreicht.

In einer Ausführungsform des erfindungsgemäßen Verfahrens sind die Schritte (4) und (5) in einem Kreislauf geschalten, wobei zumindest ein Teil des Retentates des Schrittes (4) dem Schritt (5) zugeführt wird und zumindest ein Teil des Diluates des Schrittes (5) zum Schritt (4) zurückgeführt wird.

Dadurch wird eine direkte Kreislaufführung zwischen den Schritten (4) und (5) erreicht.

In einer weiteren Ausführungsform des erfindungsgemäßen Verfahrens wird das Diluat des Schrittes (2) in einen Ausgleichsbehälter geführt, aus welchem sowohl der Schritt (4) als auch der Schritt (5) gespeist wird und in welchen zumindest ein Teil des Retentates des Schrittes (4) und ein Teil des Diluates des Schrittes (5) zurückgeführt wird.

Dadurch wird eine indirekte Kreislaufführung zwischen den Schritten (4) und (5) über den Ausgleichsbehälter erreicht.

Bevorzugt werden im erfindungsgemäßen Verfahren die Schritte (4), (5), (6) und (7), d.h. die oben beschriebene Kreislaufführung, im steady-state-Betrieb durchgeführt.

Insbesondere bevorzugt werden alle Schritte (1) bis (7) im steady-state-Betrieb durchgeführt.

Die im Schritt (1) des erfindungssgemäßen Verfahrens vorgesehene Nanofiltration ist bevorzugt eine zwei- oder mehrstufige Nanofiltration, wobei bevorzugt zumindest eine der nach der ersten Stufe durchgeführten Stufen als Diafiltration ausgebildet ist. Insbesondere können, wenn mehr als zwei Stufen vorgesehen sind, alle nach der ersten Stufe vorgesehenen Stufen als Diafiltration ausgebildet sein.

Bevorzugt wird im erfindungsgemäßen Verfahren ein Teil des Permeates des Schrittes (4) und/oder zumindest ein Teil des Diluates des Schrittes (5) der Nanofiltration im Schritt (1) zugeführt.

Insbesondere kann das gesamte Permeat des Schrittes (4) sowie jener Teil des Diluates des Schrittes (5), der gegebenenfalls nicht im Kreislauf zum Schritt. (4) geführt wird, der Nanofiltration im Schritt (1) zugeführt werden, wodurch ein praktisch geschlossener Kreislauf resultiert, aus dem lediglich die mit den Wertstoffen (A), (B) und (C) angereicherten Stoffströme ausgeschleust werden.

Wenn, wie vorzugsweise vorgesehen, die Nanofiltration zwei- oder mehrstufig durchgeführt wird, wird das Permeat des Schrittes (4) bzw. ein Teil davon und/oder der Teil des Diluates des Schrittes (5) bevorzugt der zweiten Stufe der Nanofiltration, insbesondere einer Diafiltrationsstufe zugeführt.

In der Nanofiltration des Schrittes (1) wird bevorzugt eine Membran eingesetzt, welche eine hohe Durchlässigkeit gegenüber einwertigen anorganischen Salzen und eine demgegenüber geringere Durchlässigkeit gegenüber zweiwertigen anorganischen Salzen aufweist.

Das Material der eingesetzten Membran kann bevorzugt aus der Gruppe bestehend aus permanent hydrophilisiertem Polyethersulfon, Keramik, insbesondere TiO₂, Polyamid und semiaromatischem Piperazin-Polyamid ausgewählt sein. Die Trenngrenze ("nominal molecular weight cut off" - NMWCO) liegt bei 100 bis 4000 Da, bevorzugt 100 bis 1000 Da, insbesondere bevorzugt 150 bis 300 Da.

Als Membran für das Nanofiltrationsverfahren der Stufe (1) eignen sich beispielsweise folgende Materialien:

| Hersteller | Nadir | Nadir | Koch | Nadir | Inocermic/D | Tami |
|---|---|---|---|---|---|---|
| | PES 10 | N30F | MPF36 | PES004H | Inocermic | Tami-lk |
| Material | Ph-PES | Ph-PES | Unbekannt | Ph-PES | TiO₂ | TiO₂ |
| NMWCO (Da) | 1000 | 150-350 | 1000 | 4000 | 500 | 1000 |
| Reinwasser-Permeabilität bei 20°C (Lh-¹m⁻²mpa⁻¹ | 95^{a} | 13^{a} | 60^{a} | 77^{b} | 131^{b} | 240^{b} |
| pH-Arbeitsbereich | 0-14 | 0-14 | 1-13 | 0-14 | 0-14 | 0-14 |
| Maximale Temperatur (°C) | 95 | 95 | 70 | 95 | >100 | >100 |
| Klassifikation | NF | NF | NF | UF | NF | UF |
| ph-PES: permanent hydrophilisieres Polyethersulfon; NMWCO: Nominal molecular weight cut off | | | | | | |
| ^{a} bei 1 MPa (10 bar); 20° C | | | | | | |
| ^{b} bei 0,2 MPa (2 bar); 20° C | | | | | | |

| Hersteller | GE | GE | Hydronautics | Dow |
|---|---|---|---|---|
| | Osmonics | Osmonics | NTR 7450 | Deutschland |
| | DL | DK | | NF 200 |
| Material | Polyamide | Polyamide | Ph-PES | Semi aromatic piperazine polyamide |
| NMWCO (Da) | 150-300 | 150-300 | | |
| Reinwasser-Permeabilität bei 20°C (Lh^{- 1}m⁻²MPa⁻¹ | 76 | ∼70 | 130 | 110 |
| pH-Arbeitsbereich | 2-11 | 2-11 | 2-11 | 3-10 |
| Maximale Temperatur (°C) | | | | |
| Klassifikation | NF | NF | NF | NF |

Für das Elektrodialyse-Verfahren des Schrittes (2) eignen sich insbesondere monopolare Elektrodialyseverfahren mit einem bevorzugten Transfer von Chloridionen.

Als Membranmaterial des Schrittes (2) eignen sich beipielsweise folgende Membrantypen:

| Membranhersteller | Type |
|---|---|
| Neosepta | XCMX, AMX |
| Ionics | CR69EXMP / AR103QDP |

Im Schritt (4) des erfindungsgemäßen Verfahrens werden bevorzugt dichte Umkehrosmose-Membranen in Spiralbauweise eingesetzt.

Die Umkehrosmose des Schrittes (4) wird bevorzugt unter Verwendung von hydrophilen Membran mit hoher Wasserpermeabilität bei hoher Rückhaltung von anorganischen Salzen wie Natriumchlorid durchgeführt.

Im Schritt (5) des erfindungsgemäßen Verfahrens wird bevorzugt eine monopolare Elektrodialyse durchgeführt. Bevorzugt beträgt der pH-Wert bei der Elektrodialyse zwischen 2 und 5.^{.}

Der mit dem erfindungsgemäßen Verfahren zu behandelnde Stoffstrom weist typischerweise einen pH-Wert von 1 bis 4,5 auf.

Der Wertstoff der Gruppe (A) ist bevorzugt eine oder mehrere proteinogene Aminosäure(n), z.B. Leucin.

Der Wertstoff der Gruppe (B) ist insbesondere Milchsäure.

Der Wertstoff der Gruppe (C) ist insbesondere ein oder mehrere anorganische(s) Salz(e) aus der Gruppe der Chloridsalze, insbesondere Natriumchlorid, Kaliumchlorid und/oder Mischungen daraus.

Der zu behandelnde Stoffstrom ist insbesondere eine auf einer biogenen Quelle beruhende wässrige Flüssigkeit, bevorzugt eine aus einer Silage von z.B. Gras, Klee, Luzerne, Kräutern sowie Mischungen daraus erhaltene Flüssigkeit.

Zur Durchführung des erfindungsgemäßen Verfahrens dient eine Anlage, enthaltend
- eine Nanoßltratiönsanlage 1
- eine erste Elektrodialyseanlage 2,
- eine Leitung 11 zur Überführung von Permeat der Nanofiltrationsanlage 1 zur ersten Elektrodialyseanlage 2,
- ein System 3 aus direkt oder indirekt miteinander verbundenen Aggregaten 4 und 5, wobei
- Aggregat 4 eine Umkehrosmoseanlage ist und
- Aggregat 5 eine zweite Elektrodialyseanlage ist,
- eine Leitung 21 zur Überführung von Diluat der ersten Elektrodialyseanlage 2 in das System 3,
- eine Leitung 41, mit welcher zumindest ein Teil des Retentates der Umkehrosmoseanlage 4 direkt oder indirekt zur zweiten Elektrodialyseanlage 5 geführt wird und
- eine Leitung 51, mit welcher zumindest ein Teil des Diluates der zweiten Elektrodialyseanlage direkt oder indirekt zur Umkehrosmoseanlage 4 geführt wird.

In einer Ausführungsform der erfindungsgemäßen Anlage führt die Leitung 41 direkt das Retentat der Umkehrosmoseanlage 4 der zweiten Elektrodialyseanlage 5 zu.

In einer weiteren Ausführungsform der erfindungsgemäßen Anlage führt die Leitung 51 direkt das Diluat der zweiten Elektrodialyseanlage 5 der Umkehrosmoseanlage 4 zu.

Dadurch wird eine direkte Kreislaufführung zwischen den Aggregaten 4 und 5 erreicht.

In einer alternativen Ausgestaltung der erfindungsgemäßen Anlage ist ein Ausgleichsbehälter 31 vorgesehen, in welchen die Leitung 21 für das Diluat der ersten Elektrodialyseanlage 2 mündet, aus welchem sowohl die Umkehrosmoseanlage 4 als auch die zweite Elektrodialyseanlage 5 gespeist werden und zu welchem die Leitung 41 für das Retentat der Umkehrosmoseanlage 4 und die Leitung 51 für das Diluat der zweiten Elektrodialyseanlage zurückführt.

Dadurch wird eine indirekte Kreislaufführung zwischen den Aggregaten 4 und 5 erreicht.

In der erfindungsgemäßen Anlage ist die Nanofiltrationsanlage bevorzugt mehrstufig ausgebildet, wobei besonders bevorzugt zumindest eine der nach der ersten Stufe durchgeführten Stufen als Diafiltrationsanlage ausgebildet ist.

Weiters bevorzugt ist eine Leitung 42 zur Rückführung von Permeat aus der Umkehrosmoseanlage 4 zur Nanofiltrationsanlage 1, besonders bevorzugt zu einer gegebenenfalls vorgesehenen zweiten oder weiteren Stufe der Nanofiltrationsanlage 1 vorgesehen.

Ebenfalls bevorzugt kann eine Leitung 52 zur Rückführung von Diluat aus der zweiten Elektrodialyseanlage 5 zur Nanofiltrationsanlage 1, besonders bevorzugt zur ersten Stufe der Nanofiltrationsanlage 1 vorgesehen sein.

Das erfindungsgemäße Verfahren zeichnet sich durch folgende Vorteile aus:
Jeder Teilprozess des erfindungsgemäßen Verfahrens arbeitet für sich allein gesehen mit einer Trenneffizienz (Wertstoff im Eingang zu Wertstoff im separiertem Ausgangsstrom) von 0,1 bis 0,95. Durch die Rückkopplung mit den anderen Prozessschritten kommt es aber zu Gesamtrenneffizienzen deutlich über 0,5.

Im erfindungsgemäßen Verfahren fällt insbesondere bei einer vollständigen Kreislaufführung auch des Permeats der Umkehrosmoseanlage (Schritt (4)) und des Diluates der zweiten Elektrödialyseanlage (Schritt (5)) kein Abfallstrom an.

Alle drei Produktströme ((A), (B) und (C)) sind gegenüber dem Eingangsstoffstrom aufkonzentriert.

Die Schritte der Elektrodialyse (2, 5) und der Umkehrosmose (4) beeinflussen sich gegenseitig. Eine Abtrennung von anorganischen Salzen (Schritt (2)) und eine Abtrennung von organischen Säuren (Schritt (5)) führt zu einem verringerten osmotischen Druck, was die Effizienz der Umkehrosmose im Schritt (4) erhöht. Umgekehrt führt die Aufkonzentrierung von Wertstoffen durch Umkehrosmose zu einer Effizienzsteigerung der Elektrodialyse im Schritt (5).

Umkehrosmose (4) und Nanofiltration (1) beeinflussen sich gegenseitig. Wasser aus der Umkehrosmose wird bevorzugt der zweiten Nanofiltrationsstufe beigemischt, um bevorzugt in einem Diafiltrationschritt eine Ausspülung der Wertstoffe (B) und (C) zu ermöglichen.

Nanofiltration (1) und Elektrodialyse (5) beeinflussen sich gegenseitig. Eine Nanofiltration alleine führt zu einer Anreicherung von Aminosäuren im Retentat bei teilweiser Abtrennung von organischen Säuren ins Permeat. In einer alleinigen Elektrodialyse werden organische Säuren nur unter substantiellen Verlusten an Aminosäure aus einer aminosäurenreichen Lösung getrennt. Bei dem erfindungsgemäßen Verfahren kann die Nanofiltration (1) einen großen Teil der Aminosäuren rückhalten, während die Elektrodialyse (2) kontinuierlich bei günstigen Mengenverhältnisse ((B)>>(A)) organische Säure sauber abtrennen kann. Durch die Rückführung von teilentsalztem Medium (nach den Schritten (2) und (5)) werden die Aminosäureverluste aus der Nanofiltration (1) ausgeglichen und der Milchsäuredurchsatz in einer solchen Weise erhöht, dass die Abtrennungsleistung der Elektrodialyse im Gesamtprozess gesehen einen wesentlichen Teil der Eingangsmenge der organischer Säure (B) betrifft.

Der Gesamtprozess ist zugunsten der Aminosäuren (Wertstoff (A)) ausgelegt. Es wird eine Verunreinigung des Wertstoffes (A) mit Spuren des Wertstoffes (B) in Kauf genommen, während eine maximale Ausbeute der Aminosäuren (A) angestrebt wird.

Die Erfindung wird im Folgenden anhand der Figuren und den Ausführungsbeispielen näher erläutert.

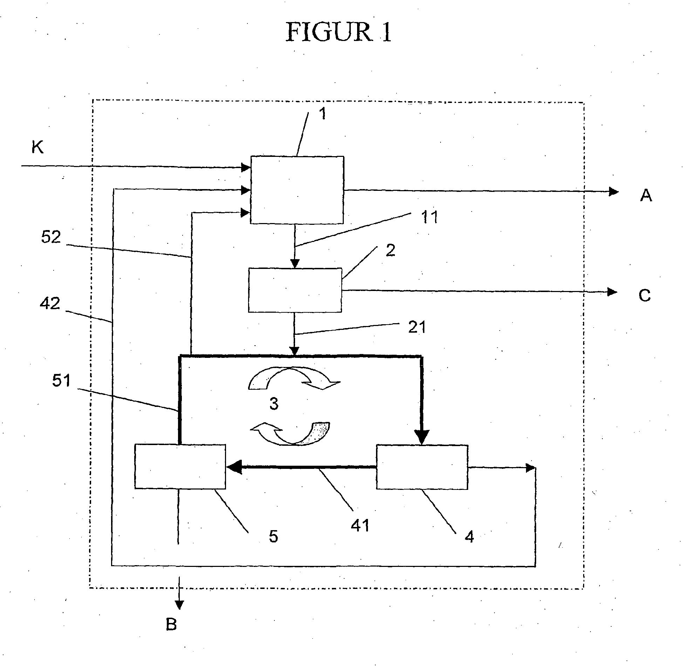

Dabei zeigt die Figur 1 schematisch den Aufbau einer Ausführungsform des erfindungsgemäßen Verfahrens und der erfindungsgemäßen Anlage.

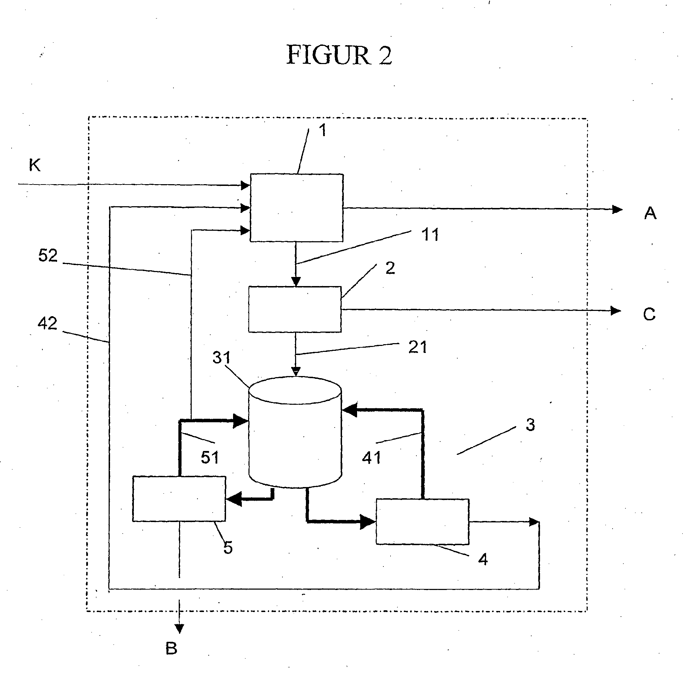

Figur 2 zeigt schematisch den Aufbau einer alternativen Ausführungsform des erfindungsgemäßen Verfahrens und der erfindungsgemäßen Anlage.

Gemäß Figur 1 wird ein komplexer Stoffstrom K einer Nanofiltrationsanlage 1 zugeführt und dort nanofiltriert. Es resultiert ein mit Aminosäuren angereichtes Retentat A, welches aus dem Verfahren ausgeschleust wird.

Das Retentat der Nanofiltration 1, welches anorganische Salze (C) und organische Säuren (B) enthält, wird über die Leitung 11 einer ersten Elektrodialyseanlage 2 zugeführt. Das Konzentrat der Elektrodialyse ist an anorganischen Salzen angereichert und wird als Werststoffstrom C ausgeschleust.

Das Diluat der ersten Elektrodialyseanlage wird über die Leitung 21 einem System 3 zugeführt, welches eine Umkehrosmoseanlage 4 und eine zweite Elektrodialyseanlage 5 beinhaltet.

Die Retentatseite der Umkehrsosmoseanlage 4 ist mittels einer Leitung 41 mit der Elektrodialyseanlage 5 verbunden. Von der Elektrodialyseanlage 5 führt diluatseitig eine Leitung 51 zur Umkehrosmoseanlage 4 zurück.

Das Konzentrat der Elektrodialyseanlage 5 ist an organischen Säuren, z.B. Milchsäure, angereichert und wird als Wertstoffstrom B ausgeschleust. Das Diluat der Elektrodialyseanlage 5 wird über die Leitung 51 zumindest zum Teil im Kreislauf zur Umkehrosmoseanlage 4 zurückgeführt. In die Leitung 51 kann beispielsweise die Zuführleitung 21 münden, wie dargestellt, die Leitung 21 kann aber auch direkt zur Umkehrosmoseanlage 4 führen.

Zumindest ein Teil des Permeates der Umkehrosmoseanlage 4 wird über die Leitung 42 der Nanofiltrationsanlage, insbesondere als Diafiltrationswasser, zugeführt.

Ebenso kann zumindest ein Teil des Diluates der zweiten Elektrodialyseanlage 5 über die Leitung 52 der Nanofiltrationsanlage 1 rückgeführt werden.

In der in Figur 2 dargestellten Ausführungsform ist ein Vorlagebehälter 31 vorgesehen, in welchen die Leitung 21 für das Diluat der ersten Elektrodialyseanlage 2 mündet.

Vom Vorlagebehälter 31 ausgehend werden sowohl die Umkehrosmoseanlage 4 als auch die Elektrodialyseanlage 5 gespeist. Das Retentat der Umkehrosmose 4 wird über die Leitung 41 in den Vorlagebehälter 31 rückgespeist. Ebenso wird zumindest ein Teil des Diluates der Elektrodialyse 5 über die Leitung 51 in den Vorlagebehälter 31 rückgespeist.

### Beispiele:

### Beispiel 1:

20 g L-Milchsäure (90 %), 30 g Kaliumlactat (50 %), 3 g L-Leucin und 10 g Kaliumchlorid werden in Wasser gelöst und auf 1 kg verdünnt.

Die so gewonnene Lösung hat folgende Konzentrationen:
27,4 g/L Milchsäure
3,0 g/L L-Leucin
11,3 g/L Kalium
4,8 g/L Chlorid

Die Lösung wird in einer Batch-Zelle bis zu einem Volumenskonzentrationsfaktor von 2 nanofiltriert. Es fallen 500 g Permeat und 500 g Konzentrat an.

Die Konzentrationen im Permeat sind:
19,2 g/L L Milchsäure
0,2 g/L L-Leucin
9,0 g/L Kalium
4,5 g/L Chlorid

Die Konzentrationen im Konzentrat sind:
35,6 g/L L Milchsäure
5,9 g/L L-Leucin
13,6 g/L Kalium
5,0 g/L Chlorid

### Beispiel 2:

20 g L-Milchsäure (90 %), 30 g Kaliumlactat (50 %), 3 g L-Glycin und 10 g Kaliumchlorid werden in Wasser gelöst und auf 1 kg verdünnt.

Die so gewonnene Lösung hat folgende Konzentrationen:
27,4 g/L Milchsäure
3,0 g/L L-Glycin
11,3 g/L Kalium
4,8 g/L Chlorid

Die Lösung wird in einer Batch-Zelle bis zu einem Volumenskonzentrationsfaktor von 2 nanofiltriert. Es fallen 500 g Permeat und 500 g Konzentrat an.

Die Konzentrationen im Permeat sind:
19,2 g/L L Milchsäure
0,9 g/L L-Glycin
9,0 g/L Kalium
4,5 g/L Chlorid

Die Konzentrationen im Konzentrat sind:
35,6 g/L L Milchsäure
5,1 g/L L-Glycin
13,6 g/L Kalium
5,0 g/L Chlorid

### Beispiel 3:

200 g L-Milchsäure (90 %), 300 g Kaliumläctat (50 %), 30 g L-Leucin und 100 g Kaliumchlorid werden in Wasser gelöst und auf 10 kg verdünnt.

Die so gewonnene Lösung hat folgende Konzentrationen:
27,4 g/L Milchsäure
3,0 g/L L-Leucin
11,3 g/L Kalium
4,8 g/L Chlorid

Die Lösung wird in einer Nanofiltrationsanlage bis zu einem Volumenskonzentrationsfaktor von 2 nanofiltriert. Es fallen 5 kg Penneat und 5 kg Konzentrat an.

Die Konzentrationen im Permeat sind:
19,2 g/L L Milchsäure
0,2 g/L L-Leucin
9,0 g/L Kalium
4,5 g/L Chlorid

Die Konzentrationen im Konzentrat sind:
35,6 g/L L Milchsäure
5,9 g/L L-Leucin
13,6 g/L Kalium
5,0 g/L Chlorid

### Beispiel 4

2 kg des Permeats von Beispiel 3 werden als Vorlage auf Feedseite einer monopolaren Elektrodialyse eingesetzt. Auf der Konzentratseite werden 1 kg Wasser vorgelegt. Bei Erreichen eines Leitfähigkeitswertes von 6,5 auf der Konzentratseite wird die Elektrodialyse gestoppt.

Die Konzentrationen feedseitig (Diluat):
18,2 g/L L Milchsäure
0,2 g/L L-Leucin
4,6 g/L Kalium
0,9 g/L Chlorid

Die Konzentrationen im Konzentrat sind:
0,9 g/L L Milchsäure
0,0 g/L L-Leucin
8,9 g/L Kalium
7,2 g/L Chlorid

### Beispiel 5 - Umkehrosmose von Diluat aus Beispiel 4:

2 kg Elektrodialysediluat aus Beispiel 4 wird in einer Umkehrosmosezelle bis zu einem Volumenskonzentrationsfaktor von 2 nanofiltriert. Es fallen 1000 g Permeat und 100 g Konzentrat an.

Die Konzentrationen im Permeat sind:
0,9 g/L L Milchsäure
0,0 g/L L-Leucin
0,2 g/L Kalium
0,1 g/L Chlorid

Die Konzentrationen im Konzentrat sind:
35,6 g/L L Milchsäure
0,4 g/L L-Leucin
9,0 g/L Kalium
1,7 g/L Chlorid

### Beispiel 6 - Elektrodialyse von Umkehrosmosekonzentrat - Extraktion der Milchsäure.

1 kg des Umkehrosmosekonzentrats von Beispiel 5 werden als Vorlage auf Feedseite einer monopolaren Elektrodialyse eingesetzt. Auf der Konzentratseite werden 1 kg Wasser vorgelegt.

Nach Transport von 67 % der Milchsäure wird der Versuch gestoppt.

Die Konzentrationen betragen feedseitig (Diluat):
11,7 g/L L Milchsäure
0,4 g/L L-Leucin
2,3 g/L Kalium
0,3 g/L Chlorid

Die Konzentrationen im Konzentrat sind:
23,8 g/L L Milchsäure
0,0 g/L L-Leucin
6,7 g/L Kalium
1,4 g/L Chlorid

### Beispiel 7

100 kg pro Stunde wässriger Lösung (K) mit einer Zusammensetzung gemäß Beispiel 1 werden in einer erfindungsgemäß aufgebauten Verfahrenskette (siehe Figur 2) behandelt.

Die Nanofiltration 1 wird als zweistufiger Prozess betrieben. Diluat aus der Elektrodialyse 5 und Umkehrosmosepermeat (Wasser) aus der Stufe 4 werden über die Leitungen 42 und 52 zur ersten Stufe der Nanofitration rückgeführt. Zusätzlich wird ein Teil des Umkehrosmosepermeates der Stufe 4 zur zweiten Stufe der Nanofiltration zurückgeführt.

Durch die Rückführungsmengen wird die Milchsäurekonzentration feedseitig eingestellt. In der ersten Stufe wird eine Milchsäurekonzentration von 25 g/L erreicht, in der zweiten Stufe eine Milchsäurekonzentration von 11 g/L.

Das Permeat aus beiden Nanofiltrationsstufen wird zusammengeführt und elektrodialysiert (2). Die Ionentransportkapazität der Elektrodialyse ist so ausgelegt, dass durch Variation der Stromstärke die abgetrennte Ionenmenge gesteuert werden kann. Es kommt zu einem bevorzugten Transport von Kaliumchlorid.

Das teilentsalzte Diluat wird in einem Behälter 31 gesammelt. Der Behälter 31 ist über Umwälzpumpen mit der Umkehrosmoseanlage 4 und der zweiten Elektrodialyseanlage 5 verbunden. Entsprechend der Permeatleistung der Umkehrosmoseanlage kommt es zu einer Aufkonzentrierung der Inhaltsstoffe. Entsprechend der Ionentransportleistung der Elektrodialyseanlage kommt es zu einer Ausschleusung von Salzen, insbesondere Kaliumlactat und Hydrogenionen.

Durch Einstellung der Permeatleistung der Umkehrosmoseanalge 4 (Steigerung durch Druckerhöhung) wird eine für die Elektrodialyse geeignete Milchsäurekonzentration von 22 g/L eingestellt.

Es wird ein Nanofiltrationskönzentratstrom (70 kg/h), angereichert mit Leucin (A) (4,2 g/kg) gewonnen.

Es wird ein Elektrodialysekonzentratstrom (20 kg/h), angereichert mit Milchsäure (B) (96 g/kg) gewonnen.

Es wird ein Elektrodialysekonzentratstrom (2 kg/h), angereichert mit Kaliumchlorid (C) (76,4 g/kg) gewonnen.

## Patentansprüche

1. Verfahren zur Behandlung eines Stoffstromes, der aus jeder der folgenden Gruppen
(A) Aminosäuren
(B) Von Aminosäuren unterschiedliche Carbonsäuren mit 1 bis 5 C-Atomen und
(C) Anorganische Salze
zumindest einen Wertstoff enthält,
welches Verfahren die folgenden Schritte aufweist:
(1) Behandlung des Stoffstromes mittels Nanofiltration, wobei ein mit Wertstoff (A) angereichertes Retentat gewonnen wird
(2) Behandlung des Permeates des Schrittes (1) mittels einer ersten Elektrodialyse, wobei ein mit Wertstoff (C) angereichertes Konzentrat gewonnen wird
(3) Behandlung des Diluates des Schrittes (2) mittels einer Umkehrosmose und einer zweiten Elektrodialyse, wobei der Umkehrosmoseschritt (4) und der zweite Elektrodialyseschritt (5) miteinander direkt oder indirekt verbunden sind,
wobei
(6) zumindest ein Teil des Retentates des Schrittes (4) direkt oder indirekt dem Schritt (5) zugeführt wird,
(7) zumindest ein Teil des Diluates des Schrittes (5) direkt oder indirekt dem Schritt (4) zugeführt wird, und
ein mit Wertstoff (B) angereichertes Konzentrat gewonnen wird.

2. Verfahren gemäß Anspruch 1, **dadurch gekennzeichnet, dass** die Schritte (4) und (5) in einem Kreislauf geschalten sind, wobei zumindest ein Teil des Retentates des Schrittes (4) dem Schritt (5) zugeführt wird und zumindest ein Teil des Diluates des Schrittes (5) zum Schritt (4) zurückgeführt wird.

3. Verfahren gemäß Anspruch 1, **dadurch gekennzeichnet, dass** das Diluat (21) des Schrittes (2) in einen Ausgleichsbehälter (31) geführt wird, aus welchem sowohl der Schritt (4) als auch der Schritt (5) gespeist wird und in welchen zumindest ein Teil des Retentates des Schrittes (4) und ein Teil des Diluates des Schrittes (5) zurückgeführt wird.

4. Verfahren gemäß einem der vorhergehenden Ansprüche, **dadurch gekennzeichnet, dass** die Schritte (4), (5), (6) und (7) im steady-state-Betrieb durchgeführt werden.

5. Verfahren gemäß Anspruch 4, **dadurch gekennzeichnet, dass** alle Schritte (1) bis (7) im steady-state-Betrieb durchgeführt werden.

6. Verfahren gemäß einem der vorhergehenden Ansprüche, **dadurch gekennzeichnet, dass** der Schritt (1) eine zwei- oder mehrstufige Nanofiltration ist, wobei bevorzugt zumindest eine der nach der ersten Stufe durchgeführten Stufen als Diafiltration ausgebildet ist.

7. Verfahren gemäß einem der vorhergehenden Ansprüche, **dadurch gekennzeichnet, dass** zumindest ein Teil des Permeats des Schrittes (4) und/oder zumindest ein Teil des Diluates des Schrittes (5) der Nanofiltration im Schritt (1) zugeführt wird.

8. Verfahren gemäß einem der vorhergehenden Ansprüche, **dadurch gekennzeichnet, dass** im Schritt (1) eine Membran eingesetzt wird, welche eine hohe Durchlässigkeit gegenüber einwertigen anorganischen Salzen und eine demgegenüber geringere Durchlässigkeit gegenüber zweiwertigen anorganischen Salzen aufweist.

9. Verfahren gemäß Anspruch 8, **dadurch gekennzeichnet, dass** das Material der eingesetzten Membran aus der Gruppe bestehend aus permanent hydrophilisiertem Polyethersulfon, Keramik, insbesondere TiO₂, Polyamid und semiaromatischem Piperazin-Polyamid ausgewählt ist.

10. Verfahren gemäß einem der vorhergehenden Ansprüche, **dadurch gekennzeichnet, dass** im Schritt (5) eine monopolare Elektrodialyse durchgeführt wird.

11. Verfahren gemäß einem der vorhergehenden Ansprüche, **dadurch gekennzeichnet, dass** der zu behandelnde Stoffstrom einen pH-Wert von 1 bis 4,5 aufweist.

12. Verfahren gemäß einem der vorhergehenden Ansprüche, **dadurch gekennzeichnet, dass** der Wertstoff der Gruppe (A) eine oder mehrere Aminosäure(n) ausgewählt aus der Gruppe bestehend aus proteinogenen Aminosäuren ist.

13. Verfahren gemäß einem der vorhergehenden Ansprüche, **dadurch gekennzeichnet, dass** der Wertstoff der Gruppe (B) Milchsäure ist.

14. Verfahren gemäß einem der vorhergehenden Ansprüche, **dadurch gekennzeichnet, dass** der Wertstoff der Gruppe (C) ein oder mehrere anorganische(s) Salz(e) aus der Gruppe der Chloddsalze, insbesondere Natriumchlorid, Kaliumchlorid und/oder Mischungen daraus ist.

15. Verfahren gemäß einem der vorhergehenden Ansprüche, **dadurch gekennzeichnet, dass** der zu behandelnde Stoffstrom eine auf einer biogenen Quelle beruhende wässrige Flüssigkeit, insbesondere eine aus einer Silage erhaltene Flüssigkeit ist.

16. Anlage zur Durchführung eines Verfahrens gemäß einem der vorhergehenden Ansprüche, enthaltend
- eine Nanofiltrationsanlage (1)
- eine erste Elektrodialyseanlage (2),
- eine Leitung (11) zur Überführung von Permeat der Nanofiltrationsanlage (1) zur ersten Elektrodialyseanlage (2)
- ein System (3) aus direkt oder indirekt miteinander verbundenen Aggregaten (4) und (5), wobei
- Aggregat (4) eine Umkehrosmoseanlage ist und
- Aggregat (5) eine zweite Elektrodialyseanlage ist,
- eine Leitung (21) zur Überführung von Diluat der ersten Elektrodialyseanlage (2) in das System (3),
- eine Leitung (41), mit welcher zumindest ein Teil des Retentates der Umkehrosmoseanlage (4) direkt oder indirekt zur zweiten Elektrodialyseanlage (5) geführt wird
- eine Leitung (51), mit welcher zumindest ein Teil des Diluates der zweiten Elektrodialyseanlage direkt oder indirekt zur Umkehrosmoseanlage (4) geführt wird.

17. Anlage gemäß Anspruch 16, **dadurch gekennzeichnet, dass** die Leitung (41) direkt das Retentat der Umkehrosmoseanlage (4) der zweiten Elektrodialyseanlage (5) zuführt.

18. Anlage gemäß Anspruch 16 oder 17, **dadurch gekennzeichnet, dass** die Leitung (51) direkt das Diluat der zweiten Elektrodialyseanlage (5) der Umkehrosmoseanlage (4) zuführt.

19. Anlage gemäß Anspruch 16, **dadurch gekennzeichnet, dass** ein Ausgleichsbehälter (31) vorgesehen ist, in welchen die Leitung (21) für das Diluat der ersten Elektrodialyseanlage (2) mündet, aus welchem sowohl die Umkehrosmoseanlage (4) als auch die zweite Elektrodialyseanlage (5) gespeist werden und zu welchem die Leitung (41) für das Retentat der Umkehrosmoseanlage (4) und die Leitung (51) für das Diluat der zweiten Elektrodialyseanlage zurückführt.

20. Anlage gemäß einem der Ansprüche 16 bis 19, **dadurch gekennzeichnet, dass** die Nanofiltrationsanlage mehrstufig ausgebildet ist, wobei bevorzugt zumindest eine der nach der ersten Stufe durchgeführten Stufen als Diafiltrationsanlage ausgebildet ist.

21. Anlage gemäß einem der Ansprüche 16 bis 20, **dadurch gekennzeichnet, dass** eine Leitung (42) zur Rückführung von Permeat aus der Umkehrosmoseanlage (4) zur Nanofiltrationsanlage (1), bevorzugt zu einer gegebenenfalls vorgesehenen zweiten Stufe oder weiteren Stufe der Nanofiltrationsanlage (1) vorgesehen ist.

22. Anlage gemäß einem der Ansprüche 16 bis 21, **dadurch gekennzeichnet, dass** eine Leitung (52) zur Rückführung von Diluat aus der zweiten Elektrodialyseanlage (5) zur Nanofiltrationsanlage (1), bevorzugt zur ersten Stufe der Nanofiltrationsanlage (1) vorgesehen ist.

## Claims

1. A process for the treatment of a stream of substances which contains at least one valuable substance from each of the following groups
(A) amino acids
(B) carboxylic acids different from amino acids and comprising from 1 to 5 C-atoms, and
(C) inorganic salts,
which process comprises the following steps:
(1) treatment of the stream of substances by nanofiltration, wherein a retentate enriched with valuable substance (A) is obtained
(2) treatment of the permeate of step (1) by a first electrodialysis, wherein a concentrate enriched with valuable substance (C) is obtained
(3) treatment of the diluate of step (2) by reverse osmosis and a second electrodialysis, wherein the step (4) of reverse osmosis and the step (5) of the second electrodialysis are directly or indirectly interconnected, wherein
(6) at least a portion of the retentate of step (4) is directly or indirectly supplied to step (5)
(7) at least a portion of the diluate of step (5) is directly or indirectly supplied to step (4), and
a concentrate enriched with valuable substance (B) is obtained.

2. A process according to claim 1, **characterized in that** the steps (4) and (5) are connected in a circuit, wherein at least a portion of the retentate of step (4) is supplied to step (5) and at least a portion of the diluate of step (5) is returned to step (4).

3. A process according to claim 1, **characterized in that** the diluate (21) of step (2) is conveyed into a balancing reservoir (31) from which both step (4) and step (5) are fed and into which at least a portion of the retentate of step (4) and a portion of the diluate of step (5) are returned.

4. A process according to any of the preceding claims, **characterized in that** the steps (4), (5), (6) and (7) are carried out in a steady-state operation mode.

5. A process according to claim 4, **characterized in that** all the steps (1) to (7) are carried out in a steady-state operation mode.

6. A process according to any of the preceding claims, **characterized in that** the step (1) is a two- or multi-stage nanofiltration, wherein preferably at least one of the stages carried out after the first stage is configured as a diafiltration.

7. A process according to any of the preceding claims, **characterized in that** at least a portion of the permeate of step (4) and/or at least a portion of the diluate of step (5) is/are supplied to the nanofiltration in step (1).

8. A process according to any of the preceding claims, **characterized in that**, in step (1), a membrane is used which exhibits a high permeability towards monovalent inorganic salts and, compared with this, a lower permeability towards divalent inorganic salts.

9. A process according to claim 8, **characterized in that** the material of the membrane used is selected from the group consisting of permanently hydrophilized polyethersulfone, ceramics, in particular TiO₂, polyamide and semiaromatic piperazine polyamide.

10. A process according to any of the preceding claims, **characterized in that**, in step (5), a monopolar electrodialysis is carried out.

11. A process according to any of the preceding claims, **characterized in that** the stream of substances to be treated has a pH-value of from 1 to 4.5.

12. A process according to any of the preceding claims, **characterized in that** the valuable substance of group (A) is one or several amino acid(s) selected from the group consisting of proteinogenic amino acids.

13. A process according to any of the preceding claims, **characterized in that** the valuable substance of group (B) is lactic acid.

14. A process according to any of the preceding claims, **characterized in that** the valuable substance of group (C) is one or several inorganic salt(s) from the group of chloride salts, in particular sodium chloride, potassium chloride and/or mixtures thereof.

15. A process according to any of the preceding claims, **characterized in that** the stream of substances to be treated is an aqueous liquid based on a biogenic source, in particular a liquid obtained from silage.

16. A plant for carrying out a process according to any of the preceding claims, comprising
- a nanofiltration installation (1)
- a first electrodialysis installation (2),
- a conduit (11) for conveying permeate from the nanofiltration installation (1) to the first electrodialysis installation (2)
- a system (3) made up of units (4) and (5) directly or indirectly connected to each other, wherein
- unit (4) is a reverse osmosis installation and
- unit (5) is a second electrodialysis installation,
- a conduit (21) for conveying diluate from the first electrodialysis installation (2) into the system (3),
- a conduit (41) by means of which at least a portion of the retentate from the reverse osmosis installation (4) is directly or indirectly conveyed to the second electrodialysis installation (5)
- a conduit (51) by means of which at least a portion of the diluate from the second electrodialysis installation is directly or indirectly conveyed to the reverse osmosis installation (4).

17. A plant according to claim 16, **characterized in that** the conduit (41) conveys the retentate from the reverse osmosis installation (4) directly to the second electrodialysis installation (5).

18. A plant according to claim 16 or 17, **characterized in that** the conduit (51) conveys the diluate from the second electrodialysis installation (5) directly to the reverse osmosis installation (4).

19. A plant according to claim 16, **characterized in that** a balancing reservoir (31) is provided into which the conduit (21) for the diluate from the first electrodialysis installation (2) runs, from which both the reverse osmosis installation (4) and the second electrodialysis installation (5) are fed and to which the conduit (41) for the retentate from the reverse osmosis installation (4) and the conduit (51) for the diluate from the second electrodialysis installation return.

20. A plant according to any of claims 16 to 19, **characterized in that** the nanofiltration installation has a multi-stage design, wherein preferably at least one of the stages carried out after the first stage is configured as a diafiltration installation.

21. A plant according to any of claims 16 to 20, **characterized in that** a conduit (42) for returning permeate from the reverse osmosis installation (4) to the nanofiltration installation (1), preferably to an optionally provided second stage or further stage of the nanofiltration installation (1), is provided.

22. A plant according to any of claims 16 to 21, **characterized in that** a conduit (52) for returning diluate from the second electrodialysis installation (5) to the nanofiltration installation (1), preferably to the first stage of the nanofiltration installation (1), is provided.

## Revendications

1. Procédé de traitement d'un flux de substance qui contient au moins une substance active de chacun des groupes suivants
(A) acides aminés
(B) divers acides carboxyliques parmi les acides aminés comprenant de 1 à 5 atomes de C, et
(C) des sels inorganiques,
lequel procédé présente les étapes suivantes :
(1) un traitement du flux de substance au moyen d'une nano filtration, un rétentat enrichi en substance active (A) étant obtenu,
(2) un traitement du perméat de l'étape 1 au moyen d'une première électrodialyse, un concentrat enrichi en substance active (C) étant obtenu,
(3) un traitement du diluat de l'étape (2) au moyen d'une osmose inverse et d'une seconde électrodialyse, l'étape d'osmose inverse (4) et l'étape de la seconde électrodialyse (5) étant reliées directement ou indirectement,
où
(6) au moins une partie du rétentat de l'étape (4) est ajoutée directement ou indirectement à l'étape (5),
(7) au moins une partie du diluat de l'étape (5) est ajoutée directement ou indirectement à l'étape (4), et
un concentrat enrichi en substance active (B) est obtenu.

2. Procédé selon la revendication 1 **caractérisé en ce que** les étapes (4) et (5) sont effectuées en boucles, au moins une partie du rétentat de l'étape (4) étant ajoutée à l'étape (5) et au moins une partie du diluat de l'étape (5) étant ajoutée à l'étape (4).

3. Procédé selon la revendication 1 **caractérisé en ce que** le diluat (21) de l'étape (2)) est réalisé dans un récipient de compensation (31) à partir duquel, à la fois l'étape (4) et l'étape (5), sont alimentées et dans lequel au moins une partie du rétentat de l'étape (4) et une partie du diluat de l'étape (5) sont recyclées.

4. Procédé selon l'une des revendications précédentes **caractérisé en ce que** les étapes (4), (5), (6) et (7) sont effectuées en fonctionnement continu.

5. Procédé selon la revendication 4 **caractérisé en ce que** les étapes (1) à (7) sont effectuées en fonctionnement continu.

6. Procédé selon l'une des revendications précédentes **caractérisé en ce que** l'étape (1) est une nano filtration à deux ou à plusieurs étages, de préférence, au moins une des étapes réalisée après le premier étage étant conçue sous la forme d'une diafiltration.

7. Procédé selon l'une des revendications précédentes **caractérisé en ce qu'**au moins une partie du perméat de l'étape (4) et/ou au moins une partie du diluat de l'étape (5) est ajoutée à la nano filtration de l'étape (1).

8. Procédé selon l'une des revendications précédentes **caractérisé en ce que**, dans l'étape (1), on emploie une membrane qui présente une forte perméabilité vis-à-vis des sels inorganiques monovalents et une perméabilité moindre à celle-ci par rapport aux sels inorganiques divalents.

9. Procédé selon la revendication 8 **caractérisé en ce que** le matériau de la membrane employé est choisi dans le groupe constitué par le polyéther sulfone hydrophylisé, la céramique, en particulier TiO₂, le polyamide et un polyamide-pipérazine semi aromatique.

10. Procédé selon l'une des revendications précédentes, **caractérisé en ce que**, dans l'étape (5), on effectue une électrodialyse mono polaire.

11. Procédé selon l'une des revendications précédentes **caractérisé en ce que** le flux de substance à traiter présente une valeur de pH entre 1 et 4,5.

12. Procédé selon l'une des revendications précédentes **caractérisé en ce que** la substance active du groupe (A) fait partie du groupe d'un ou de plusieurs acide(s) aminé(s) choisis dans le groupe constitué par des acides aminés protéinogènes.

13. Procédé selon l'une des revendications précédentes **caractérisé en ce que** la substance active fait partie du groupe (B) des acides lactiques.

14. Procédé selon l'une des revendications précédentes **caractérisé en ce que** la substance active fait partie du groupe (C) d'un ou de plusieurs sel(s) inorganique(s) constitué par les sels chlorures, en particulier, le chlorure de sodium, le chlorure de calcium et/ou des mélanges de ceux-ci.

15. Procédé selon l'une des revendications précédentes **caractérisé en ce que** le flux de substance à traiter est un liquide aqueux basé sur une source aqueuse biogène calmante, en particulier, est un liquide obtenu par un ensilage.

16. Installation destinée à l'exécution d'un procédé selon l'une des revendications précédentes, comprenant
- une installation de nano filtration (1),
- une première installation d'électrodialyse (2),
- une canalisation (11) pour le transfert des perméats de l'installation de nano filtration (1) vers la première installation d'électrodialyse (2),
- un système (3) constitué d'unités (4) et (5) reliées entre elles directement ou indirectement, où
- l'unité (4) est une installation d'osmose inverse et
- l'unité (5) est une deuxième installation d'électrodialyse,
- une canalisation (21) pour le transfert de diluat de la première installation d'électrodialyse (2) dans le système (3),
- une canalisation (41) par laquelle au moins une partie du rétentat de l'installation d'osmose inverse (4) est menée directement ou indirectement vers la deuxième installation d'électrodialyse (5),
- une canalisation (51) par laquelle au moins une partie du diluat de la deuxième installation d'électrodialyse est menée directement ou indirectement vers l'installation d'osmose inverse (4).

17. Installation selon la revendication 16 **caractérisée en ce que** la canalisation (41) mène le rétentat de l'installation de l'osmose inverse (4) directement à la deuxième installation d'électrodialyse (5).

18. Installation selon les revendications 16 ou 17 **caractérisée en ce que** la canalisation (51) mène le diluat de la deuxième installation d'électrodialyse (5) directement à l'installation d'osmose inverse (4).

19. Installation selon la revendication 16 **caractérisée en ce qu'**un récipient de compensation (31) est prévu, récipient dans lequel débouche la canalisation (21) pour le diluat de la première installation d'électrodialyse (2), à partir duquel à la fois l'installation d'osmose inverse (4) et la deuxième installation d'électrodialyse (5) sont alimentées et vers lequel la canalisation (41) pour le rétentat de l'installation d'osmose inverse (4) et la canalisation (51) pour le diluat de la deuxième installation d'électrodialyse retournent.

20. Installation selon l'une des revendications de 16 à 19 **caractérisée en ce que** l'installation de nano filtration est conçue sous la forme de plusieurs étages, où de préférence au moins une étape parmi les étapes réalisées après la première étape est conçue comme une installation de diafiltration.

21. Installation selon l'une des revendications de 16 à 20 **caractérisée en ce qu'**une canalisation (42) est prévue pour le recyclage de perméat à partir de l'installation d'osmose inverse (4) vers l'installation de nano filtration (1), de préférence à une éventuelle seconde étape prévue ou à une autre étape de l'installation de nano filtration (1).

22. Installation selon l'une des revendications de 16 à 21 **caractérisée en ce qu'**une canalisation (52) est prévue pour le recyclage de diluat provenant de la deuxième installation d'électrodialyse (5) vers l'installation de nano filtration (1), de préférence à une première étape de l'installation de nano filtration (1).
